# EUROPEAN PATENT SPECIFICATION

(11) **EP 1 621 147 B1**
(45) Date of publication and mention of the grant of the patent: **28.03.2007**
(21) Application number: 04254559.0
(22) Date of filing: 29.07.2004
(51) Int. Cl.: A61B 17/12

(54) **Band ligator for surgical treatment**
Chirurgische Vorrichtung zur Gummibandligatur
Ligateur chirurgical aux bandes élastiques

(43) Date of publication of application: 01.02.2006
(73) Proprietor: Taewoong Medical Co., Ltd., Koyang-si, Kyunggi-do 411-360 (KR); Shin, Kyong-Min, Seoul 157-765 (KR)
(72) Inventor: Shin, Kyong-min, Seoul 157-765 (KR); Lee, Jong-taek, Seoul 120-826 (KR)
(74) Representative: Fenlon, Christine Lesley

(56) References cited:
- WO-A-97/45060
- US-A- 5 398 844
- US-A- 5 853 416
- US-A1- 2004 006 256

## Description

The present invention relates generally to band ligators, and more particularly, to a band ligator for medical treatment, which is inserted along with ligating bands into a patient's body using an endoscope toward lesions, on which, for example, weak portions of a blood vessel protrude, or which are bleeding, or which comprise one or more cysts, so that the lesions are ligated by the ligating bands, and thus, nectrotized.

Generally, when weak portions of blood vessels protrude from parts of patients' bodies, such as the walls of the esophagus, or when portions of the walls of the esophagus bleed, the patients may be in danger because of bleeding from the above-mentioned lesions.

Typically, the above-mentioned lesions are treated by thermotherapies, such as electric cauterization, laser cauterization, or heat irradiation therapy, etc., or by drug therapies using injections. However, the conventional therapies may injure or affect healthy tissues of the patients around the lesions.

To avoid the above-mentioned problems of conventional therapies, recently, band ligators have been used. The conventional band ligators supply ligating bands to lesions of patients' bodies using endoscopes and ligate the lesions, on which weak portions of blood vessels protrude, or which are bleeding. Thus, the lesions are necrotized by poor blood circulation. As such, the conventional band ligators can naturally treat the lesions.

The above-mentioned conventional therapies using the band ligators have been efficiently used to treat the lesions on which weak portions of the blood vessel protrude, or which bleed, or which are various cysts.

The detailed explanation of the conventional therapies using the band ligators is as follows.

The conventional band ligators are mounted on typical endoscopes with flexible tubes. The conventional band ligators each include a main barrel which is a front end of the flexible tube of the endoscope, with a plurality of ligating bands wound around an outer surface of the main barrel. The conventional band ligator further includes a controller which is provided on a main body of the endoscope and controls the band ligator to sequentially supply each of the ligating bands wound around the main barrel to each of the lesions, thus ligating the lesions.

The above-mentioned endoscope has a plurality of channels. For example, the endoscope may have an illumination channel to transmit light to the distal end of the tube of the endoscope, and an air suction channel to draw air into the endoscope through the tube.

The conventional band ligator further has a thread which is woven through the ligating bands wound around the main barrel to connect the ligating bands to the controller through the interior of the main barrel and the flexible tube of the endoscope.

To use the conventional band ligator, a user places the main barrel of the band ligator at the lesion site using the endoscope while observing the main barrel through the endoscope. Thereafter, the lesion is drawn into the main barrel by an air suction action of the endoscope through the air suction channel.

In the above state, each of the ligating bands wound around the main barrel is supplied to each of the lesions by the thread operated by the controller. Then, the ligating band, which has been expanded, is contracted to ligate the lesion. Thus, the lesion is necrotized by the poor circulation of blood.

After the above-mentioned operation of ligating one lesion is completed, the user moves the main barrel of the band ligator toward other lesions without removing the endoscope from the patient body. Thereafter, other lesions are continuously treated in the same manner as that of the above-mentioned ligating operation. As described above, the conventional band ligator has a structure which is capable of sequentially supplying the ligating bands wound around the main barrel to the lesions. Therefore, several ligating operations can be executed in one session.

Various methods have been proposed and used to sequentially supply the ligating bands, wound around the outer surface of the main barrel, to the lesions.

A band ligator is disclosed in US Patent No. 6,007,551 and includes a thread which passes under a plurality of ligating bands wound around an outer surface of a main barrel of the band ligator. The thread is coupled to a leading wire through an endoscope. A part of the thread, placed in each of the spaces defined between ligating bands, should have a sufficient length to correspond to a distance extending from each of the ligating bands to a distal end of the main barrel. The band ligator of No. 6,007,551 further includes a bead which is provided on the thread in back of each of the ligating bands to be in close contact with the ligating band.

In the band ligator of No. 6,007,551, the first bead pushes the first ligating band forwards as the thread is taken up (pulled). The length of the thread between the first ligating band and the second ligating band is sufficient to ensure that the second ligating band is not moved until the first ligating band is completely removed from the main barrel of the band ligator.

After the first ligating band is supplied to a first lesion by the thread being pulled, the second and third ligating bands are sequentially supplied to second and third lesions by continuously :pulling the thread, thus ligating the lesions.

However, in the band ligator of No. 6,007,551 having the above-mentioned structure, the plurality of beads must be mounted on the thread, thus causing inconvenience to the user. Furthermore, the supply of the ligating bands to the lesions is accomplished by contact with the beads. At this time, the beads may be undesirably removed from the thread. In addition, it is very difficult to arrange the thread appropriately between the ligating bands such that movement of a subsequent ligating band is limited until a former ligating band is completely removed from the main barrel.

If the length of the thread placed between the ligating bands is incorrect, or the bead is undesirably removed from the thread, the ligating band cannot be smoothly applied to the lesion.

A band ligator is also disclosed in US Patent No. 5,398,844 and includes a plurality of threads which are respectively wound around a plurality of ligating bands provided around an outer surface of a main barrel of the band ligator. These threads are coupled to a leading wire through an endoscope.

In the band ligator of No. 5,398,844, the ligating bands are sequentially supplied to lesions as the threads, wound around the ligating bands, are sequentially pulled.

However, the band ligator of No. 5,398,844 requires the user to wind each of the threads around each of the ligating bands, thus causing inconvenience to the user. Furthermore, the threads may become undesirably entangled with each other. In addition, the user may pull the wires in the wrong order, thus causing an operation error.

A further band ligator is disclosed in WO97/45060. In this arrangement a plurality of ligating bands are carried on a support means, and the ligating bands are pulled off the distal end of the support means by a displacement element or thread. The displacement element is looped around each ligating band and a respective pair of adjacent notches in the distal end of the support means. This document is consided to represent the closest prior art.

Accordingly, preferred embodiments of the present invention have been made keeping in mind the above problems occurring in the prior art, and seek to provide a band ligator for a medical treatment, in which a ring-shaped thread is woven through a plurality of ligating bands, so that the ligating bands are sequentially and efficiently taken up by a drum of a controller.

According to the present invention there is provided a band ligator for a medical treatment, comprising a main barrel provided on a distal end of a flexible tube of an endoscope, with a through hole provided in the main barrel; a plurality of ligating bands provided around an outer surface of the main barrel; and a thread which is woven through the ligating bands and is operable to be proximally pulled in order to sequentially move the ligating bands towards a distal end of the main barrel, and further comprising:
a plurality of left and right notches provided on a circumferential edge of the distal end of the main barrel, each left notch being provided at a substantially equal distance from a reference point on the circumferential surface of the barrel as a corresponding right notch,
wherein both ends of the thread are connected to each other to form a loop which is caught between a first left notch and a corresponding right notch,
wherein each side of the thread extends from the left or right notch respectively and is woven through the successive ligating bands and the successive notches on the respective left or right side of the barrel so as to bind the litigating bands together such that the ligating bands are operable to be sequentially expelled from the distal end of the barrel.

Preferred features of the invention are the subject of the dependent claims.

For a better understanding of the present invention, and to show how the same may be carried into effect, reference will now be made, by way of example to the accompanying drawings in which:
FIG. 1 is a front view of an endoscope having a band ligator according to an embodiment of the present invention;
FIG. 2 is a partially broken sectional view of a controller of the band ligator of FIG. 1;
FIG. 3 is a partially broken sectional view of a main barrel of the band ligator of FIG. 1;
FIG. 4 is a side view of the main barrel of FIG. 3;
FIGS. 5a through 5d are views showing an operation of weaving a thread through a plurality of ligating bands wound around the main barrel of Fig. 3; and
FIG. 6 is a view showing an operation of the band ligator of FIG. 1.

Reference now should be made to the drawings, in which the same reference numerals are used throughout the different drawings to designate the same or similar components.

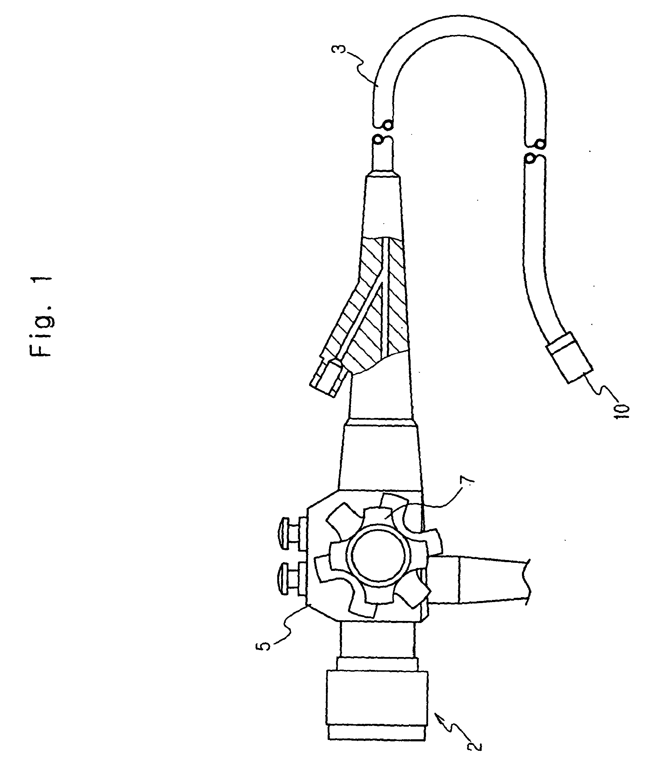

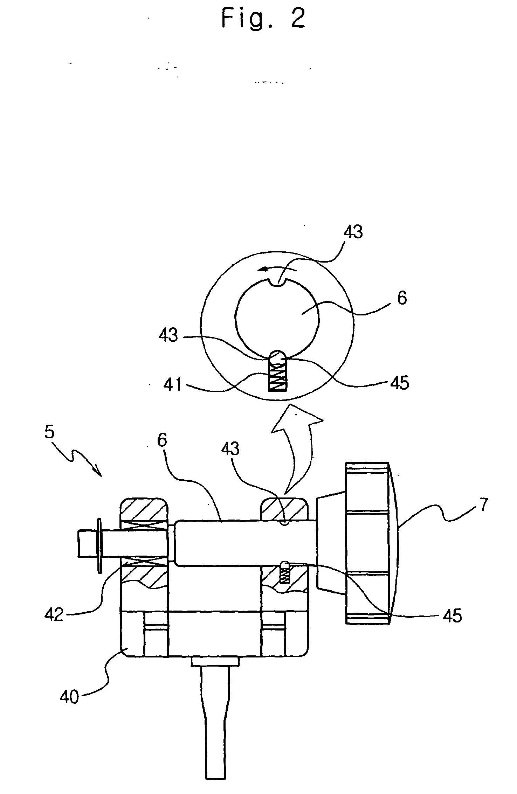

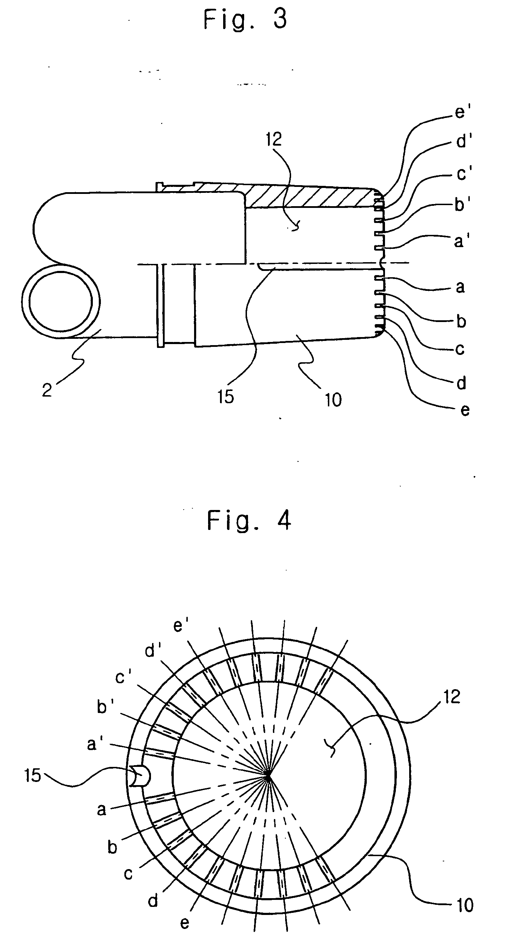

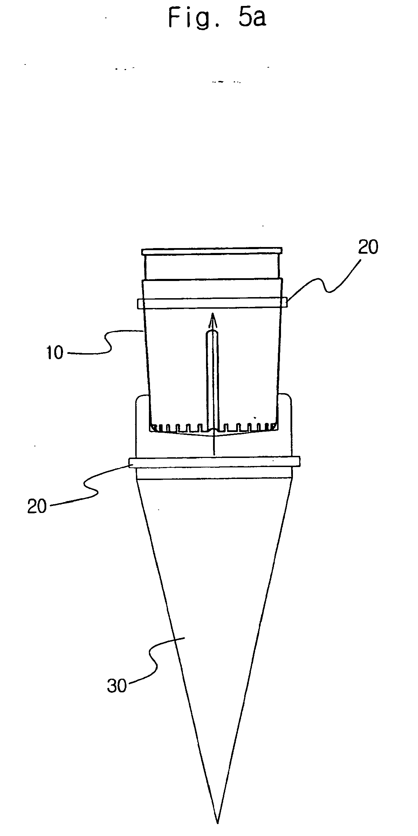

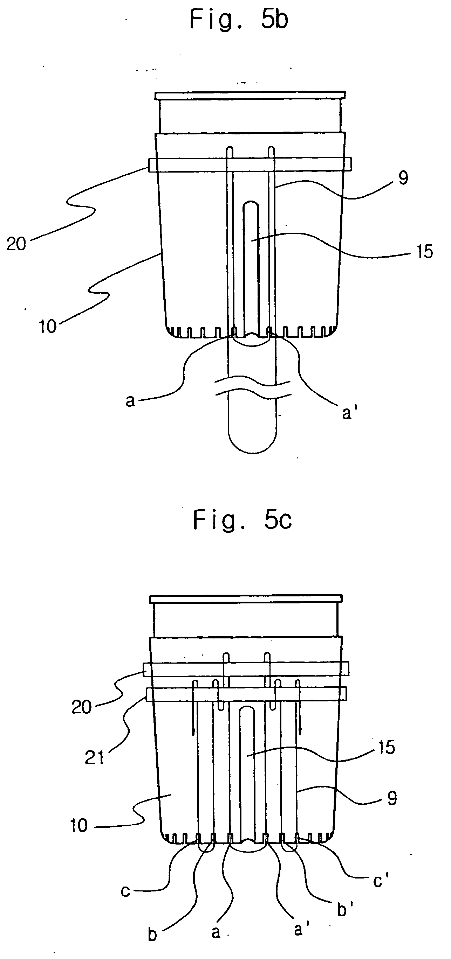

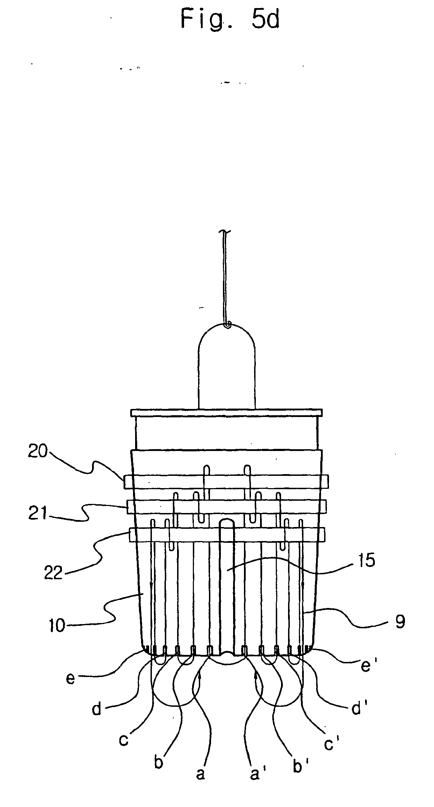

As shown in FIGS. 1 through 5d, a band ligator for a medical treatment according to an embodiment of the present invention includes a main barrel 10 which is provided on a distal end of a flexible tube 3 of an endoscope 2, with a through hole 12 provided in the main barrel 10. The band ligator further includes a plurality of ligating bands 20, 21 and 22 which are provided around an outer surface of the main barrel 10. The band ligator further includes a ring-shaped or looped thread 9 which is woven through the ligating bands 20, 21 and 22 and is wound around a drum 6 of a controller 5 while being guided to the outside through the flexible tube 3. The ligating bands are sequentially moved through an end of the main barrel 10 to lesions as the ring-shaped thread 9 is taken up by the drum 6 in response to operation of a handle 7 of the controller 5, thus ligating the lesions.

The band ligator further includes a plurality of left and right hanging notches a, b, c, d, e and a', b', c', d', e' which are provided on opposite parts of a circumferential edge of the distal end of the main barrel 10 to be symmetric to each other.

The thread 9 is connected at both ends thereof to each other so as to form a loop. A predetermined portion of the looped thread 9 is caught by the first left and right hanging notches a and a'. Opposite parts of the ring-shaped thread 9 individually pass under a first ligating band 20 rearwards prior to passing over the first ligating band 20 forwards, thus binding the first ligating band 20. Thereafter, the parts of the ring-shaped thread 9 also individually pass under a second ligating band 21 forwards. The parts of the ring-shaped thread 9 thereafter pass over the second ligating band 21 rearwards prior to passing under the second ligating band 21 forwards, until the parts of the ring-shaped thread 9 are caught by the second and third left and right hanging notches b, c, b' and c', thus binding the second ligating band 21.

Thereafter, the parts of the ring-shaped thread 9 individually pass under a third ligating band 22 forwards. The parts of the ring-shaped thread 9 thereafter pass over the third ligating band 22 rearwards prior to passing under the third ligating band 22 forwards, until the parts of the ring-shaped thread 9 are caught by the fourth and fifth left and right hanging notches d, e, d' and e', thus binding the third ligating band 22. Thereafter, the ring-shaped thread 9 may continuously bind the remaining ligating bands together in the same manner as that of binding the third ligating band 22.

Preferably, the ligating bands 20, 21 and 22 may be wound around the outer surface of the main barrel 10 in groups of five or ten.

A band ligator embodying the present invention further includes a groove 15 which is provided on the outer surface of the main barrel 10 at a position between the plurality of left and right hanging notches a, b, c, d, e and a', b', c', d' e'. The groove 15 allows a user to hold up the ligating bands 20, 21 and 22 from the outer surface of the main barrel 10, thus easily weaving the ring-shaped thread 9 through the ligating bands 20, 21 and 22.

The drum 6 wound with the ring-shaped thread 9 is installed in the controller 5 to be rotated in one direction by a unidirectional bearing 42 on a base 40. At this time, the ring-shaped thread 9 is directly wound around the drum 6 or is coupled to a leading wire which is wound around the drum 6. A plurality of seat slots 43 is provided on predetermined portions of a circumferential outer surface of the drum 6 to be spaced apart from each other at regular intervals. The base has a locking hole 41 on a predetermined position thereof to correspond to the seat slots 43. A locking ball 45 is elastically installed in the locking hole 41. Thus, each of the ligating bands 20, 21 and 22 is supplied to each of the lesions as the ring-shaped thread 9 is taken up by the drum 6 while the locking ball 45 is elastically seated from a seat slot 43 of the drum 6 into another seat slot 43 by a rotation of the drum 6.

In the drawings, the reference numeral 30 denotes a band guide 30 to guide the ligating bands 20, 21 and 22 to the outer surface of the main barrel 10.

The operation and effect of the band ligator of the present invention will be described herein below.

To easily wind the ligating bands 20, 21 and 22 around the main barrel 10, the main barrel 10 is coupled to the band guide 30 having a conical shape.

In the above state, the ligating bands 20, 21 and 22 are fitted over the outer surface of the main barrel 10 while being elastically expanded through the band guide 30.

Thereafter, the ring-shaped thread 9 is woven through the ligating bands 20, 21 and 22 wound around the outer surface of the main barrel 10, thus sequentially moving the ligating bands 20, 21 and 22.

In a detailed description, the first ligating band 20 is fitted over the outer surface of the main barrel 10. Thereafter, the predetermined portion of the ring-shaped thread is caught by the first left and right hanging notches a and a'. In the above state, the opposite parts of the ring-shaped thread 9 individually pass under the first ligating band 20 rearward prior to passing over the first ligating band 20 forwards, thus binding the first ligating band 20.

At this time, the ring-shaped thread 9 can easily pass under the first ligating band 20 rearwards, while a bar (not shown) is inserted into the groove 15 of the main barrel 10 to hold up the first ligating band 20.

After the first ligating band 20 is bound with the ring-shaped thread 9 around the main barrel 10, the second ligating band 21 is fitted over the outer surface of the main barrel 10 in front of the first ligating band 20 in the same manner as that of fitting the first ligating band 20 over the outer surface of the main barrel 10.

At this time, the ring-shaped thread 9 is placed on the main barrel 10 such that the opposite parts of the ring-shaped thread 9 individually pass under the second ligating band 21 forwards.

In the above state, the opposite parts of the ring-shaped thread 9 individually pass over the second ligating band 21 rearwards prior to passing under the second ligating band 21 forwards, until the opposite parts of the thread 9 are caught by the second and third left and right hanging notches b, c, b' and c', thus binging the second ligating band 21.

After the second ligating band 21 is wound around the main barrel 10, the third ligating band 22 is fitted over the outer surface of the main barrel 10 in front of the second ligating band 21 in the same manner as that of fitting the first or second ligating band 20 or 21 over the main barrel 10. At this time, the ring-shaped thread 9 is placed on the main barrel 10 such that the opposite parts of the ring-shaped thread 9 individually pass under the third ligating band 22 forwards.

In the above state, the opposite parts of the ring-shaped thread 9 individually pass over the third ligating band 22 rearwards prior to passing under the third ligating band 22 forwards, until the opposite parts of the ring-shaped thread 9 are caught by the fourth and fifth left and right hanging notches d and e and d' and e', thus binding the third ligating band 22. The second and third ligating bands 21 and 22 are bound by the ring-shaped thread 9 in the same manner as each other. The ring-shaped thread 9 may continuously bind the remaining ligating bands together in the same manner as that of binding the second or third ligating bands 21 or 22.

Preferably, the ligating bands 20, 21 and 22 are wound around the outer surface of the main barrel 10 in groups of five or ten.

While the ring-shaped thread 9 is woven through the ligating bands 20, 21 and 22, the ring-shaped thread 9 may not be sequentially caught by the left and right hanging notches a, b, c, d, e and a', b', c', d' e'. In other words, the ring-shaped thread 9 may be caught by the hanging notches in an overlapping manner. However, in this case, the ring-shaped thread 9 may be undesirably entangled. Therefore, the ring-shaped thread 9 is preferably caught by the hanging notches a, b, c, d, e and a', b', c', d' e' sequentially.

To treat lesions of a patient using the band ligator embodying the present invention, the main barrel 10, around which the ligating bands 20, 21 and 22 are bound by the ring-shaped thread 9, is coupled to the distal end of the flexible tube 3 of the endoscope 2.

The other predetermined part of the ring-shaped thread 9 woven through the ligating bands 20, 21 and 22 is wound around the drum 6 after being guided to the outside through the flexible tube 3.

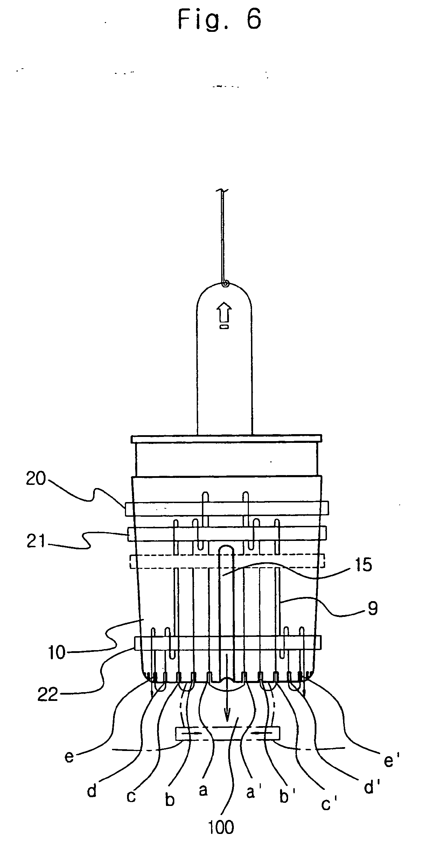

In the above state, the distal end of the main barrel 10 is placed near a first lesion 100 of a patient' body using the endoscope 2. Thereafter, the first lesion 100, such as a protruded part of a blood vessel, or a cyst, etc., is sucked into the through hole 12 of the main barrel 10 by an air suction operation of the endoscope 2, as shown in FIG. 6.

The handle 7 of the controller 5 is thereafter rotated.

Thus, the drum 6 rotates one click until the locking ball 45 is elastically seated from a seat slot 43 into another seat slot 43.

During the above-mentioned one-click rotation of the drum 6, the ring-shaped thread 9 is taken up by the drum 6 to a predetermined length, so that the third ligating band 22, which is the frontmost ligating band, is supplied to the first lesion 100.

That is, because the ring-shaped thread 9 is woven through the third ligating band 22, the third ligating band 22 moves forwards while the ring-shaped thread 9 is taken up by the drum 6 to the predetermined length.

At this time, the second ligating band 21, which is next to the third ligating band 22, does not move. In a detailed description, the opposite parts of the ring-shaped thread 9 individually pass over the third ligating band 22 rearwards prior to passing under the third ligating band 22 forwards until the parts of the ring-shaped thread 9 are caught by the fourth and fifth left and right hanging notches d and e and d' and e'. Furthermore, the ring-shaped thread 9 has a sufficient spare length (L), so that the second ligating band 22 does not move while the third ligating band 22 bound by the ring-shaped thread 9 moves forwards as the ring-shaped thread 9 is taken up by the drum 6.

The spare length (L) of the ring-shaped thread 9 is not set by the user, but is naturally obtained while the ring-shaped thread 9 is woven through the ligating bands 20, 21 and 22 and the left and right hanging notches a, b, c, d, e and a', b', c', d', e'. Therefore, a process of assembling the band ligator is simplified. In addition, the ring-shaped thread 9 can be woven through the ligating bands 20, 21 and 22 without causing an error in the spare length of the ring-shaped thread 9.

Moreover, the thread 9 has a ring-shaped appearance. Therefore, when the ring-shaped thread 9 is taken up by the drum 6, the opposite parts of the ring-shaped thread 9 simultaneously pull left and right parts of each of the ligating bands 20, 21 and 22 on the main barrel 10. Thus, each of the ligating bands 20, 21 and 22 evenly moves forwards on the outer surface of the main barrel 10.

In other words, if a piece of thread is woven through the ligating bands 20, 21 and 22, the thread pulls only a predetermined part of each of the ligating bands 20, 21 and 22. Therefore, the ligating bands 20, 21 and 22 may not smoothly move forwards, and thus, may not be completely removed from the main barrel 10. To solve the above-mentioned problems, a plurality of threads may be used for binding the ligating bands 20, 21 and 22. However, if the threads are used, the process of binding the ligating bands 20, 21 and 22 is very complicated. To alleviate the above-mentioned problems, the ring-shaped thread 9 is used in the band ligator of the present invention.

As such, the ligator band 22 is supplied to the first lesion 100 after being removed from the main barrel 10 by the ring-shaped thread 9. Thereafter, the ligator band 22 is contracted on the first lesion 100, thus necrotizing the first lesion 100 by poor blood circulation.

After the first lesion 100 is treated through the above-mentioned treatment process, the main barrel 10 is promptly moved by the endoscope 2 toward another lesion. Thereafter, the band ligator embodying the present invention invention repeatedly executes the above mentioned treatment process.

That is, in the band ligator of the present invention, the plurality of ligating bands 20, 21 and 22 are wound around the outer surface of the main barrel 10. The ligating bands 20, 21 and 22 are sequentially supplied to the lesions. Therefore, the band ligator embodying the present invention can advantageously treat the several lesions in one operation.

When the band ligator treats another lesion, the drum 6 rotates one click by the operation of the handle 7 of the controller 5 until the locking ball 45 is elastically seated from the seat slot 43 into another seat slot 43.

By the above-mentioned one-click rotation of the drum 6, the ring-shaped thread 9 or the leading wire coupled to the ring-shaped thread 9 is wound around the drum 6. Then, the ring-shaped thread 9 pulls the second ligating band 21 next to the third ligating band 22. Thus, the second ligating band 21 is supplied to another lesion, thus ligating the lesion.

As such, the ligatin bands 20, 21 and 22 are sequentially supplied to the lesions by the operation of the handle 7 of the controller 5 until the locking ball 45 is elastically seated from the seat slot 43 into another seat slot 43. Therefore, the user can advantageously execute the surgical operation even though the user cannot observe the lesions with the naked eye.

Furthermore, in the band ligator of the present invention, the unidirectional bearing 42 prevents the drum 6 from reversely rotating. Therefore, the ring-shaped thread 9 is precisely taken up by the drum 6 in response to the operation of the handle 7 of the controller 5, thus preventing an operational error.

As described above, embodiments of the present invention provide a band ligator for a medical treatment, in which a ring-shaped thread is simply woven through a plurality of ligating bands wound around an outer surface of a main barrel, so that the ligating bands are sequentially and efficiently supplied to lesions as the thread is pulled by a drum of a controller.
Furthermore, when the ring-shaped thread is pulled by the drum, opposite parts of the ring-shaped thread simultaneously pull left and right parts of each of the ligating bands. Thus, the ligating bands are efficiently supplied to the lesions. In addition, even when additional process of controlling the spare length of the ring-shaped thread is not necessary to sequentially supply the ligating bands to the lesions without an operational error, thus enhancing the reliability of products.

Although the preferred embodiments of the present invention have been disclosed for illustrative purposes, those skilled in the art will appreciate that various modifications, additions and substitutions are possible, without departing from the scope of the claims.

## Claims

1. A band ligator for a medical treatment, comprising a main barrel (10) provided on a distal end.of a flexible tube (3) of an endoscope, with a through hole provided in the main barrel; a plurality of ligating bands (20, 21, 22) provided around an outer surface of the main barrel; and a thread (9) which is woven through the ligating bands and is operable to be proximally pulled in order to sequentially move the ligating bands towards a distal end of the main barrel, the ligator further comprising:
a plurality of left and right notches (a, b, c, d, e, a', b', c', d', e') provided on a circumferential edge of the distal end of the main barrel, each left notch being provided at a substantially equal distance from a reference point on the circumferential surface of the barrel as a corresponding right notch,
wherein both ends of the thread are connected to each other to form a loop which is caught between a first left notch (a) and a corresponding first right notch (a'),
wherein each side of the thread extends from the left or right notch respectively and is woven through the successive ligating bands (20 etc) and the successive notches (b, c; b', c' etc) on the respective left or right side of the barrel so as to bind the tigating bands together such that the ligating bands are operable to be sequentially expelled from the distal end of the barrel.

2. A band ligator as claimed in claim 1, wherein each side of the thread passes under a first ligating band (20) rearwards prior to passing over the first ligating band forwards, thus binding the first ligating band, and then passes under a second ligating band (21) forwards, and passes over the second ligating band rearwards prior to passing under the second ligating band forwards, until the parts of the thread are caught by the second (b, b') and third (c, c') left and right notches, thus binding the second ligating band, and
the parts of the thread individually pass under a third ligating band (22) forwards, and pass over the third ligating band rearwards prior to passing under the third ligating band forwards, until the parts of the thread are caught by the fourth (d, d') and fifth (e, e') left and right notches, thus binding the third ligating band, and wherein the thread continuously binds the remaining litigating bands together in the same manner as that of binding the third ligating band.

3. The band ligator according to claim 1, or 2, further comprising:
a groove provided on the outer surface of the main barrel at said reference point between the plurality of left and right notches to allow a user to hold up the ligating bands to be spaced apart from the outer surface of the main barrel.

## Patentansprüche

1. Bandligator für eine medizinische Behandlung, umfassend
eine Hauptpinole (10) am entfernten Ende des flexiblen Rohrs (3) eines Endoskops, welche ein Durchgangsloch aufweist;
eine Anzahl Ligaturbänder (20, 21, 22), welche um die Außenwand der Hauptpinole angeordnet sind; und
ein Faden (9), der durch die Ligaturbänder gewebt ist und so fahrbar ist, dass er herangezogen werden kann, um nach und nach die Ligaturbänder zum entfernten Ende der Hauptpinole zu ziehen;
wobei der Ligator zudem aufweist:
eine Anzahl linker und rechter Kerben (a, b, c, d, e, a', b', c', d', e'), die bereitgestellt sind auf der Kreisumfangskante des entfernten Endes der Hauptpinole, wobei die linken Kerben jeweils mit im Wesentlichen gleichen Abstand zu einem Bezugspunkt auf der Kreisumfangsfläche der Pinole wie die entsprechenden rechten Kerben,
wobei beide Enden des Fadens miteinander verbunden sind und eine Schlaufe bilden, die zwischen einer ersten linken Kerbe (a) und einer entsprechenden ersten rechten Kerbe (a') gefangen ist,
wobei die Enden des Fadens jeweils weglaufen von der linken bzw. der rechten Kerbe und gewoben sind durch die folgenden Ligaturbänder (20, etc.), und die folgenden Kerben (b, c; b', c', etc.) auf der linken bzw. der rechten Seite der Pinole, so dass die Ligaturbänder zusammen gebunden werden derart, dass die Ligaturbänder geführt werden können um nacheinander vom entfernten Ende der Pinole ausgegeben zu werden.

2. Bandligator nach Anspruch 1, wobei die Enden des Fadens jeweils unter einem ersten Ligaturband (20) nach hinten laufen bevor sie über das erste Ligaturband nach vorne laufen, wodurch das erste Ligaturband gebunden wird, und dann unter einem zweiten Ligaturband (21) nach vorne laufen und über das zweite Ligaturband nach hinten bevor sie unter dem zweiten Ligaturband nach vorne laufen bis die Teile des Fadens gefangen sind von der zweiten (b, b') und der dritten (c, c') linken und rechten Kerbe, wodurch das zweite Ligaturband gebunden wird, und
die Teile des Fadens einzeln unter einem dritten Ligaturband (22) nach vorne laufen und über das dritte Ligaturband nach hinten, bevor sie unter dem dritten Ligaturband nach vorne laufen, bis die Teile des Fadens von der vierten (d, d') und der fünften (e, e') linken und rechten Kerbe gefangen sind, wodurch das dritte Ligaturband gebunden wird, und wobei der Faden stetig die verbleibenden Ligaturbänder in gleicher Weise zusammenbindet wie beim Binden des dritten Ligaturbandes.

3. Bandligator nach Anspruch 1 oder 2, zudem umfassend auf der Außenwand der Hauptpinole eine Kerbe am Bezugspunkt zwischen der Anzahl linker und rechter Kerben, so dass ein Anwender die Ligaturbänder, die von der Außenwand der Hauptpinole zu beabstanden sind, hochhalten kann.

## Revendications

1. Dispositif de ligature de bande pour traitement médical, comprenant un tambour principal (10) prévu à une extrémité distale d'un tube flexible (3) d'un endoscope, avec un trou traversant prévu dans le tambour principal ; une pluralité de bandes de ligature (20, 21, 22) prévues autour d'une surface extérieure du tambour principal ; et un fil (9) qui est tissé dans les bandes de ligature et qui peut être actionné pour être tiré du côté proximal, pour déplacer les bandes de ligature en séquence vers une extrémité distale du tambour principal, le dispositif de ligature comprenant par ailleurs :
une pluralité d'encoches gauches et droites (a, b, c, d, e, a', b', c', d', e') prévues sur un bord circonférentiel de l'extrémité distale du tambour principal, chaque encoche gauche étant prévue à une distance sensiblement égale d'un point de référence sur la surface circonférentielle du tambour qu'une encoche droite correspondante,
dans lequel les deux extrémités du fil sont connectées l'une à l'autre, pour former une boucle qui est saisie entre une première encoche gauche (a) et une encoche droite correspondante (a'),
dans lequel chaque côté du fil s'étend respectivement à partir de l'encoche gauche ou droite et est tissé à travers les bandes de ligature successives (20, etc.) et des encoches successives (b, c ; b', c', etc.) du côté gauche ou droit respectif du tambour, de manière à lier entre elles les bandes de ligature de sorte que les bandes de ligature puissent être actionnées pour être éloignées en séquence de l'extrémité distale du tambour.

2. Dispositif de ligature de bande selon la revendication 1, dans lequel chaque côté du fil passe sous une première bande de ligature (20) vers l'arrière, avant de passer au-dessus de la première bande de ligature vers l'avant, liant ainsi la première bande de ligature, et passe ensuite sous une deuxième bande de ligature (21) vers l'avant, et passe au-dessus de la deuxième bande de ligature vers l'arrière, avant de passer sous la deuxième bande de ligature vers l'avant, jusqu'à ce que les parties du fil soient saisies par les deuxième (b, b') et troisième (c, c') encoches gauches et droites, liant ainsi la deuxième bande de ligature, et
les parties du fil passent individuellement sous une troisième bande de ligature (22) vers l'avant, et passent au-dessus de la troisième bande de ligature vers l'arrière, avant de passer sous la troisième bande de ligature vers l'avant, jusqu'à ce que les parties du fil soient saisies par les quatrièmes (d, d') et cinquièmes (e, e') encoches gauches et droites, liant ainsi la troisième bande de ligature, et dans lequel le fil lie en continu les entre elles les bandes de ligature restantes de la même manière que pour lier la troisième bande de ligature.

3. Dispositif de ligature de bande selon la revendication 1 ou 2, comprenant par ailleurs :
une rainure prévue sur la surface extérieure du tambour principal audit point de référence entre la pluralité d'encoches gauches et droites, pour permettre à un utilisateur de retenir les bandes de ligature à écarter de la surface extérieure du tambour principal.
